# EUROPEAN PATENT SPECIFICATION

(11) **EP 1 366 754 B1**
(45) Date of publication and mention of the grant of the patent: **29.03.2006**
(21) Application number: 03011495.3
(22) Date of filing: 21.05.2003
(51) Int. Cl.: A61Q 5/10

(54) **Composition for the dyeing of human hair**
Zusammensetzung zum Färben von menschlichen Haaren
Composition pour la coloration des cheveux humains

(30) Priority: 28.05.2002 DE 10223559; 28.05.2002 DE 20208254 U
(43) Date of publication of application: 03.12.2003
(73) Proprietor: KPSS-Kao Professional Salon Services GmbH, 64297 Darmstadt (DE)
(72) Inventor: Nöcker, Bernd, Dr., 64372 Ober-Ramstadt (DE); Vega-Carrascal, Isabel, 47104 Valladolid (ES)

(56) References cited:
- EP-A- 0 801 942
- EP-A- 1 127 566
- EP-A- 1 269 975
- DE-A- 10 114 545
- DE-U- 20 003 105
- DE-U- 20 011 145
- DE-U- 20 100 131
- DE-U- 20 100 857
- DE-U- 20 105 305
- DE-U- 20 115 893
- US-A- 5 942 216

## Description

This invention is about hair dyeing composition for human hair on aqueous basis and in an emulsion form comprising at least one direct-acting hair anionic dyestuff, an emulsifier and has a pH in the range of 1 to 5.

Such hair dyeing compositions have been known for some time.

They mostly contain several anionic or cationic direct-acting dyestuffs and, in difference to the permanent hair dyeing compositions on the basis of oxidation dyestuff precursors, they require no prior mixing with oxidizing agents.

These direct-acting compositions are either applied onto the hair together with surface-active substances as so-called tinting shampoos for a moderate dyeing intensity or, applied to hair, as lotions, emulsions or thickened solutions, i.e. gels for intensive colorations.

The pH-value of these hair dying compositions on the basis of direct-acting dyestuffs generally ranges between about 3 and about 7.

Until recently as direct-acting dyestuffs it is customary to use cationic direct-acting hair dyestuffs, although it is basically known and possible to use anionic dyestuffs for achieving intensive colorations.

In practice number of difficulties are observed when coloring hair with direct acting acidic dyestuff containing dyeing agents. One of them and the most important one is the skin coloring which occurs simultaneously during hair dyeing process. The known and also commercially available coloring compositions based on direct acting acidic dyes, show very intensive and brilliant coloration on the hair and very disadvantageous in marketing such products. However, the undesired skin tinting is also found to be extremely high. Thus, the need is there for newly developed coloring agents based on acidic direct dyestuffs showing intensive, brilliant coloring effect on hair, but without or, if not avoidable, at very low level skin tinting side effect. The invention, thus, consists of formulating hair coloring agents for avoiding undesired excessive skin coloration.

DE 201 05 305 U1 discloses colour giving shampoo compositions. DE 201 00 131 U1 is on hair colouring compositions based on oxidative dyes and cationic or neutral direct dyes. EP 801 942 A1 is on hair colouring compositions with cationic direct dyes. EP 1269975 A2 and DE 200 03 105 U1 discloses hair colouring composition comprising direct dyes, which can as well be anionic dyes. None of the documents mentioned here disclose an emulsion composition comprising anionic dyes.

The document **DE 200 11 145 U1** discloses a hair dyeing composition on aqueous basis comprising at least one direct-acting anionic dyestuff having a pH of maximum 2.5. Said hair dyeing compositions can be in *emulsion form*.

Surprisingly, it has now been found out that by using emulsions it is possible to avoid, or reduce, such undesired excessive skin coloring, which is, if present, then also easier to remove. Water based coloring emulsion of the present invention comprises, in addition to direct acting anionic (acidic) dyes, emulsifiers selected from anionic, nonionic, cationic and zwitterionic or amphoteric character, and one or more fatty alcohols of the formula

R₁₃-OH

where R₁₃ is a saturated or unsaturated, branched or non-branched fatty acyl chain with 10 - 24 C atoms and has a pH in the range from 1.0 to 5.0. preferably 1.5 to 4.5, more preferably 1.5 to 3,5.

Hair dyeing emulsion composition of the present invention shows lower skin staining when tested under the conditions disclosed with the example 1 of the description (see further under the examples to illustrate the invention) when compared to coloring agents containing acidic dyes thickened only with polymers and are not in the from of emulsion.

The anionic (acidic) direct-acting hair dyestuffs are customarily incorporated in amounts from about 0.005 % to about 5 %, preferably about 0.01 % to about 3.5 %, more preferably about 0.1 % to about 2.5% and most preferably 0.1 - 1.0% by weight, calculated to the total composition.

Suitable anionic dyestuffs are, for example:

| | |
|---|---|
| Acid Black 1, | C.I.-No. 20,470; |
| Acid Blue 1, | C.I.-No. 42,045; |
| Food Blue 5, | C.I.-No. 42,051; |
| Acid Blue 9, | C.I.-No. 42,090; |
| Acid Blue 74, | C.I.-No. 73,015; |
| Acid Red 18, | C.I.-No. 16,255; |
| Acid Red 27, | C.I.-No. 16,185; |
| Acid Red 87, | C.I.-No. 45,380; |
| Acid Red 92, | C.I.-No. 45,410; |
| Acid Orange 7, | C.I.-No. 15,510; |
| Acid Violet 43, | C.I.-No. 60,730; |
| Acid Yellow 1, | C.I.-No. 10,316; |
| Acid Yellow 23, | C.I.-No. 19,140; |
| Acid Yellow 3, | C.I.-No. 47,005; |
| Food Yellow No. 8, | C.I.-No. 14,270; |
| D&C Brown No. 1, | C.I.-No. 20,170 |
| D&C Green No. 5, | C.I.-No. 61,570; |
| D&C Orange No. 4, | C.I.-No. 15,510; |
| D&C Orange No. 10, | C.I.-No 45,425:1; |
| D&C Orange No. 11, | C.I.-No. 45,425; |
| D&C Red No. 21, | C.I.-No. 45,380:2; |
| D&C Red No. 27, | C.I.-No. 45,410:1; |
| D&C Red No. 33, | C.I.-No. 17,200; |
| D&C Yellow No. 7, | C.I.-No. 45,350:1; |
| D&C Yellow No. 8, | C.I.-No. 45,350; |
| FD&C Red No. 4, | C.I.-No. 14,700; |
| FD&C Yellow No. 6, | C.I.-No. 15,985. |

Plant dyestuffs can also be used alone or in combination with synthetic direct-acting dyestuffs, for example henna (red or black), alkanna root, laccaic acid, indigo, logwood powder, madder root and rhubarb powder, etc.

Organic or inorganic acid or their mixtures are added to adjust the pH-value of compositions to the desired value. Examples to organic acids are citric acid, lactic acid, tartaric acid, malic acid, maleic acid, benzoic acid, fumaric acid, levulinic acid, butyric acid, valeric acid, oxalic acid, succinic acid, mandelic acid, glycolic acid or inorganic acids such as hydrochloric acid, phosphoric acid, sulphuric acid, nitric acid. Concentration of the organic or inorganic acids and their mixtures should be adjusted in a way that composition has a pH value between 1 and 5. The pH of the composition can also be adjusted to the required pH by using an alkaline solution such as sodium hydroxide, potassium hydroxide in the case that at the selected acid concentration pH of the composition is lower than that of the aimed value.

The hair dyeing composition according to the invention comprises surface-active substances as emulsifiers. These can be anionic and/or nonionic and/or cationic and/or amphoteric or zwitterionic and/or their mixtures.

Preferred are nonionic and/or anionic surfactants or their mixtures as emulsifiers in an amount ranging between 0.1 - 10 %, preferably 0.5 - 5% by weight calculated to the total composition.

Suitable nonionic surfactants as emulsifier are compounds from the category of alkyl polyglucosides with the general formula

R₁-O-(CH₂CH₂O)ₙ-Zₓ

wherein R₁ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fafty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Other additionally useful non-ionic surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Additionally useful surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain. Suitable amineoxides are on the market, for example, under the trade names "Ammonyx®", "Aromox®" or "Genaminox®".

Further suitable surfactant components are fatty acid mono- and dialkanolamides, such as coco fatty acid monoethanolamide and myristic fatty acid monoisopropanolamide.

Anionic surfactants can also be part of the composition of the present invention as emulsifiers. Those are of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Suitable surfactants of the carboxylate type are alkyl polyether carboxylic acids and the salts thereof of the formula

R₂-(C₂H₄O)ₙ-O-CH₂COOX,

wherein R₂ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 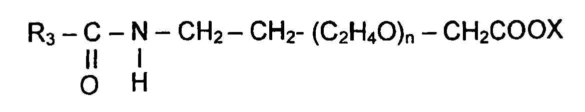 wherein R₃ and X have the above meanings, and n is in particular a number from 1 to 10, preferably 2.5 to 5. Such products have been known and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

An overview of the anionic surfactants can furthermore be found in the monography of K. Schrader, "Grundlagen und Rezepturen der Kosmetika", 2^{nd} Ed.(1989, Hüthig Buchverlag), pp. 595-600 and pp. 683 to 691. Those are mainly used in liquid body cleansing compositions, and at the same time especially suitable for coloring compositions of the present invention as emulsifiers.

Suitable amphoteric or zwitterionic surfactants are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 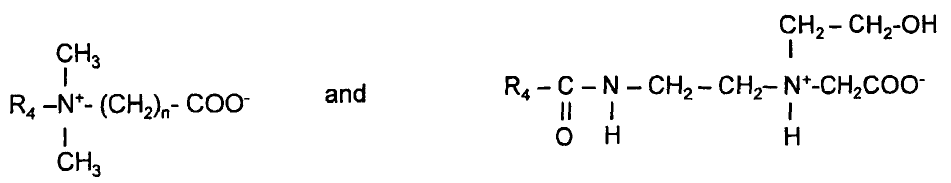 wherein R₄ is a C₈-C₁₈-alkyl group and n is 1 to 3,
sulfobetaines of the structure 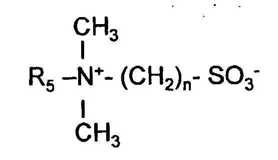 wherein R₅ is a C₈-C₁₈-alkyl group and n is 1 to 3,
and amido alkyl betaines of the structure, 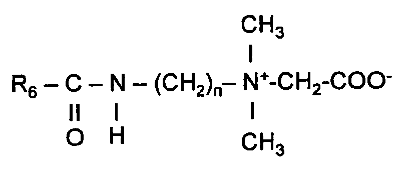 wherein R₆ is a C₈-C₁₈-alkyl group and n is 1 to 3.

Preferred are fatty acid amidoalkyl betaines, in particular cocoamidopropyl betaine, and cocoamphoacetate and -propionate, in particular the sodium salts thereof.

Concentration of those amphoteric or zwitterionic surfactants used as emulsifiers can be in the range of 0.1 - 5%, preferably 0.1 - 2.5% and more preferably 0.1 -1 1% by weight calculated to the total composition.

Cationic surfactants (as emulsifiers) and/or hair conditioning agents can also be part of the coloring compositions of the present invention which are represented with the general formula below: 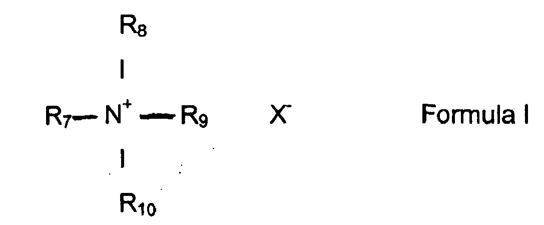 where R₇ is a saturated or unsaturated, branched or non-branched alkyl chain with 8-22 C atoms,
or

R₁₁ CO NH (CH₂)n

where R₁₁ is saturated or unsaturated, branched or non-branched alkyl chain with 7-21 C atoms and n has typical value of 0 - 4,
or

R₁₂ CO O (CH₂)n

where R₁₂ is saturated or unsaturated, branched or non-branched alkyl chain with 7-21 C atoms and n has typical value of 0 - 4 or

R₈ is H or unsaturated or saturated, branched or non-branched alkyl chain with 1-22 C atoms or

R₁₁ CO NH (CH₂)n

or

R₁₂ CO O (CH₂)ₙ

where R₁₁, R₁₂ and n are same as above.

R₉ and R₁₀ are independent from each other H or lower alkyl chain with 1 to 4 Carbon atoms, and X is chloride, bromide, methosulfate.

Suitable cationic surfactants and or conditioning agents are, for example, long-chain quaternary ammonium compounds which can be used alone or in admixture with one another, such as cetyl trimethyl ammonium chloride, dimethyl diethyl ammonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate.

Concentration of those cationic surfactants and/or conditioning agents vary typically from 0.1 - 5%, preferably 0.1 - 2.5% and more preferably 0.1 - 1% by weight calculated to the total composition.

The coloring composition of the present invention contains fatty alcohols with the general formula

R₁₃-OH

where R₁₃ is a saturated or unsaturated, branched or non-branched fatty acyl chain with 10 - 24 C atoms. Concentration of fatty alcohols is usually less than 20%, preferably less than 15% and more preferably less than 10% by weight calculated to total composition. Typical example to the most useful fatty alcohols are myristyl alcohol, palmityl alcohol, cetyl alcohol, And the most preferred one is the cetearyl alcohol.

Of special importance here are solvents which are used as solubilizers and at the same time which enhances penetration of the anionic dyes into the hair which is necessary in order to achieve intensive long lasting colorations. These "penetration enhancers" are, for example, benzyloxyethanol, benzyl alcohol, phenoxy ethanol, phenoxy isopropanol, methyl phenoxy ethanol, benzyl glycerol, N-benzyl formide, benzyl urea, N-methyl pyrrolidone, N-ethyl pyrrolidone, cinnamyl alcohol, phenethyl-alcohol, p-methyl benzyl alcohol, butyl cellosolve, methyl carbitol, ethyl carbitol, propyl carbitol, butyl carbitol, diethylene glycol, diethyl ether and dipropylene glycol diethyl ether.

Concentration of those solvents is at least 5%, preferably at least 7.5% and more preferably at least 10% by weight calculated to total composition. In any case the solvent content of the composition of the present invention should not exceed 35%, preferably 30% and more preferably 25% by weight calculated to the total composition.

The viscosity of the compositions according to the invention preferably ranges from 1,000 to 80,000, preferably from 5,000 to 70,000, more preferably from 5,000 to 60,000 mPa.s and most preferably from 10,000 to 60,000 measured at 20°C, with a Brookfield rotation viscosimeter with for example spindle no. 5 at 5 rpm.

These compositions can also contain further conditioning agents such as fats and oils.

These are, for example, sun flower oil, almond oil, peach kernel oil, wheat germ oil, macadamia nut oil, night primrose oil, jojoba oil, castor oil, or also olive or soya oil, lanolin and the derivatives thereof, as well as mineral oils such as paraffin oil and petrolatum.

Synthetic oils and waxes are, for example, silicone oils, polyethylene glycols, etc.

Further suitable hydrophobic components are in particular fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters such as PEG-7-glyceryl cocoate, cetyl palmitate, etc..

The hair dyeing compositions according to the invention may also contain thickening agents though those should not be used as the main thickener. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof, synthetic polymers such as the various polyacrylic acids, etc., in amounts from about 0.1 % to about 2 % by weight, calculated to the total composition and depending on the desired consistency thereof.

The compositions according to the invention can also comprise long-chain fatty acids. Preferred fatty acids are those with 10 to 24, in particular 12 to 22 carbon atoms, in an amount from about 0.5 % to 15 % by weight, in particular 1 % to 10 % by weight, in reference to the total composition. Especially suited are behenic acid and stearic acid, however, it is also possible to incorporate other fatty acids such as, for example, myristic acid, palmitic acid or oleic acid, or also mixtures of natural or synthetic fatty acids such as coco fatty acid.

The following examples are to illustrate the invention.

| **Example 1: Gel (comparative)** | | **Example 1a: Emulsion** | |
|---|---|---|---|
| Acid Red 52 | 0.5 % by zt. wt. | Acid Red 52 | 0.5 % by wt. |
| Ethanol | 15.0 | Ethanol | 15.0 |
| Benzyloxyethanol | 5.0 | Benzyloxyethanol | 5.0 |
| Xanthan Gum | 1.5 | Cetearyl alcohol | 13.5 |
| Sodium hydroxide (32%) | 0.25 | Sodium cetearyl sulfate | 1.5 |
| Phosphoric acid | q.s pH 2.0 | Ceteareth-20 | 5.0 |
| Water ad | 100.0 | Phosphoric acid | q.s. pH 2.0 |
| | | Water ad | 100.0 |

Example 1 is prepared by dissolving all ingredients in the given order in water.

Example 1a is prepared by melting first cetearyl alcohol, sodium cetearyl sulfate and ceteareth-20 in part of the water at 80°C and other ingredients are dissolved in 80°C water. Afterwards both portions are combined and emulsified with the aid of homogenizer at for example 2000 rpm. The composition is cooled down and pH is adjusted. Viscosity of the emulsion at 20°C was 48,000 mPa.s measured with a Brookfield viscosimeter, spindle 4 at 5 rpm.

Coloring is carried out on goat and human hair (color level 8) at 50°C for 20 min. Afterwards the swatches are rinsed with water and shampooed once with a commercial shampoo. For skin staining, the tests are carried out on forearm at room temperature, approximately 20°C, for 20, min and rinsed off with water. For washing tests with shampoo commercial shampoo is used. For testing the efficiency of the color stain remover, as well available commercially under the brand name Goldwell, the stain remover is first absorbed into a cotton and the stain is tried to be rubbed by the cotton.

For calculation of Delta E values, color difference before and after coloration or washing or rubbing, L, a and b values are measured with a commercial equipment and Delta E values are calculated with the known mathematical equation.

| | **Example 1** | **Example 1a** |
|---|---|---|
| Coloration of goat hair (ΔE) | 74.05 | 74.36 |
| Coloration of human hair (ΔE) | 33.47 | 34.33 |
| Coloration of the skin after 1 minute rinsing with water (ΔE) | 72.37 | 57.06 |
| Coloration of the skin after 1 minute washing with shampoo (ΔE) | 65.90 | 42.00 |
| Coloration of the skin after 20-fold rubbing with a cotton cloth moistened with stain remover (ΔE) | 44.66 | 23.06 |
| Coloration of the skin after 50-fold rubbing with a cotton cloth moistened with stain remover (ΔE) | 25.41 | 6.28 |

| **Example 2: Gel (comparative)** | | **Example 2a: Emulsion** | |
|---|---|---|---|
| Acid Violet 43 | 0.3 % by wt. | Acid Violet 43 | 0.3 % by wt. |
| Ethanol | 15.0 | Ethanol | 15.0 |
| Benzyl alcohol | 1.0 | Benzyl alcohol | 1.0 |
| Xanthan Gum | 1.8 | Cetearyl alcohol | 13.5 |
| Sodium hydroxide (32%) | 0.25 | Sodium cetearyl sulfate | 1.5 |
| Phosphoric acid ad | pH 2.0 | Ceteareth-20 | 5.0 |
| Water ad | 100.0 | Phosphoric acid ad | pH 2.0 |
| | | Water ad | 100,0 |

Preparation and all measurement processes are same as in the example 1. The viscosity of the emulsion is 55000 mPa.s measured at 20°C with a Brookfield viscosimeter spindle 4, at 5 rpm.

| | **Example 2** | **Example 2a** |
|---|---|---|
| Coloration of goat hair (ΔE) | 58.54 | 65.58 |
| Coloration of human hair (ΔE) | 24.70 | 33.63 |
| Coloration of the skin after 1 minute rinsing with water (ΔE) | 35.06 | 6.35 |
| Coloration of the skin after 1 minute washing with shampoo (ΔE) | 28.34 | 5.02 |
| Coloration of the skin after 20-fold rubbing with a cotton cloth moistened with stain remover (ΔE) | 10.29 | 2.89 |
| Coloration of the skin after 50-fold rubbing with a cotton cloth moistened with stain remover (ΔE) | 6.78 | 2.98 |

| **Example 3:Gel (comparative)** | | **Example 3a: Emulsion** | |
|---|---|---|---|
| Acid Red 52 | 0.5 % by wt. | Acid Red 52 | 0.5 % by wt. |
| Ethanol | 15.0 | Ethanol | 15.0 |
| Benzyl alcohol | 1.0 | Benzyl alcohol | 1.0 |
| Xanthan Gum | 1.8 | Cetearyl alcohol | 11.25 |
| Sodium hydroxide (32%) | 0.25 | Ceteareth-20 | 3.75 |
| Phosphoric acid ad pH | 2.0 | Phosphoric acid ad pH | 2.0 |
| Water ad | 100.0 | Water ad | 100.0 |

Preparation and all measurement processes are same as in the example 1. The viscosity of the emulsion is 45000 mPa.s measured at 20°C with a Brookfield viscosimeter spindle 4, at 5 rpm.

| | **Example 3** | **Example 3a** |
|---|---|---|
| Coloration of goat hair (dE) | 70.56 | 73.46 |
| Coloration of human hair (dE) | 35.89 | 33.01 |
| Coloration of the skin after 1 minute rinsing with water | 72.03 | 53.68 |
| Coloration of the skin after one minute washing with shampoo | 52.87 | 27.93 |
| Coloration of the skin after 20-fold rubbing with a cotton cloth moistened with stain remover | 34.81 | 14.95 |
| Coloration of the skin after 50-fold rubbing with a cotton cloth moistened with stain remover | 16.65 | 8.71 |

| **Example 4:Gel (comparative)** | | **Example 4a: Emulsion** | |
|---|---|---|---|
| Acid Red 52 | 0.3 % by wt. | Acid Red 52 | 0.3 % by wt. |
| Ethanol | 15.0 | Ethanol | 15.0 |
| Benzyloxyethanol | 5.0 | Benzyl alcohol | 1.0 |
| Xanthan Gum | 1.5 | Cetearyl alcohol | 13.5 |
| Sodium hydroxide (32%) | 0.25 | Sodium cetearyl sulfate | 1.5 |
| Phosphoric acid ad pH | 2.0 | Ceteareth-20 | 5.0 |
| Water | ad 100.0 | Phosphoric acid ad pH | 2.0 |
| | | Water | ad 100.0 |

Preparation and all measurement processes are same as in the example 1. The viscosity of the emulsion is 50000 mPa.s measured at 20°C with a Brookfield viscosimeter spindle 4, at 5 rpm.

| | **Example 4** | **Example 4a** |
|---|---|---|
| Coloration of goat hair (ΔE) | 67.13 | 68.58 |
| Coloration of human hair (ΔE) | 31.06 | 33.63 |
| Coloration of the skin after 1 minute rinsing with water (ΔE) | 47.5 | 13.98 |
| Coloration of the skin after 1 minute washing with shampoo (ΔE) | 40.46 | 10.74 |
| Coloration of the skin after 20-fiold rubbing with a cotton cloth moistened with stain remover (ΔE) | 29.86 | 8.67 |
| Coloration of the skin after 50-fold rubbing with a cotton cloth moistened with stain remover (ΔE) | 20.58 | 4.48 |

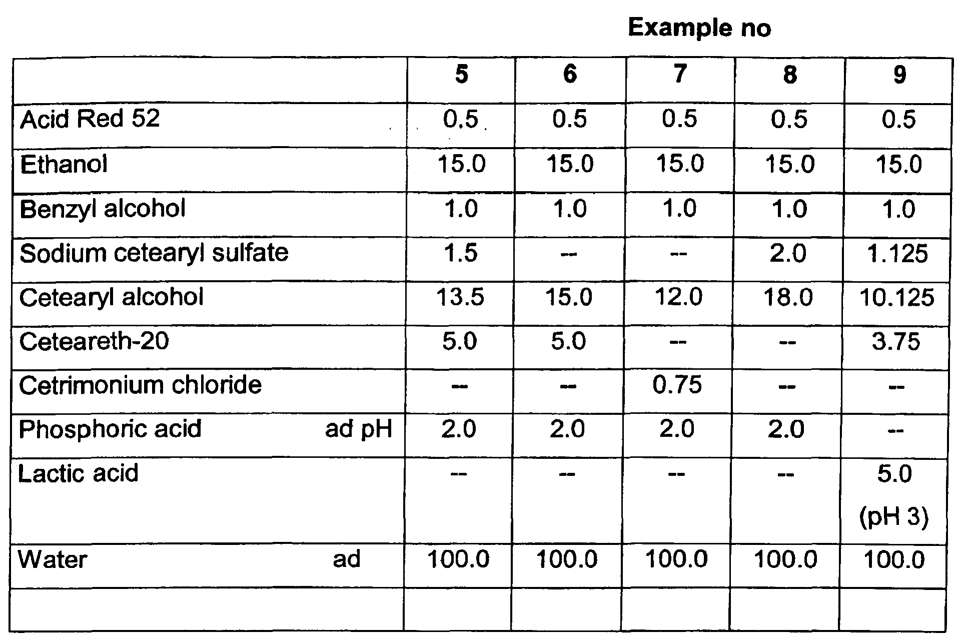

All values in the examples 5 - 9 are given as weight %.

Examples are produced in the same way as in the example 1 and all tests are carried out as in the same way as in the example 1.

| | **5** | **6** | **7** | **8** | **9** |
|---|---|---|---|---|---|
| Coloration of goat hair (ΔE) | 75.31 | 75.02 | 74.40 | 72.25 | 73.44 |
| Coloration of human hair (ΔE) | 32.84 | 36.59 | 34.65 | 32.53 | 29.10 |
| Coloration of the skin after 1 minute rinsing with water (ΔE) | 57.68 | 65.56 | 65.11 | 66.86 | 46.63 |
| Coloration of the skin after 1 minute washing with shampoo (ΔE) | 36.43 | 39.47 | 46.34 | 40.01 | 17.01 |
| Coloration of the skin after 20-fold rubbing with cotton cloth moistened with stain remover (ΔE) | 13.02 | 10.74 | 13.78 | 16.62 | 12.20 |
| Coloration of the skin after 50-fold rubbing with cotton cloth moistened with stain remover (ΔE) | 3.47 | 3.37 | 4.96 | 3.41 | 10.11 |

### Example 10:

| | | |
|---|---|---|
| Acid Violet 43 | | 0.30 % by wt. |
| Acid Black 1 | | 0.30 |
| D&C Orange 4 | | 0.20 |
| D&C Red 33 | | 0.20 |
| Alcohol, den. | | 15.00 |
| Benzyloxyethanol | | 1.00 |
| Cetearyl alcohol | | 13.50 |
| Sodium cetearyl sulfate | | 1.50 |
| Ceteareth-20 | | 5.00 |
| Lactic acid | | 4.50 |
| Dimethicone copolyol | | 1.00 |
| Perfume | | 0.20 |
| Phosphoric acid | ad pH | 2.0 |
| Water | ad | 100.0 |

A glossy, black-blue colored hair was obtained, which was easy to comb and showed full texture and elasticity. The skin coloration was simple to remove by means of rubbing with a cotton cloth moistened with a stain remover.

### Example 11:

| | | |
|---|---|---|
| Acid Violet 43 | | 0.30 % by wt. |
| Acid Black 1 | | 0.30 |
| D&C Orange 4 | | 0.20 |
| D&C Red 33 | | 0.20 |
| Cetearyl alcohol | | 10.125 |
| Sodium cetearyl sulfate | | 1.125 |
| Ceteareth-20 | | 3.75 |
| Benzyloxyethanol | | 5.00 |
| Alcohol, den. | | 10.00 |
| Benzyl carbamate | | 1.70 |
| Hydroxyethyl cellulose | | 1.50 |
| Sodium hydroxide | | 0.25 |
| Dimethicone copolyol | | 1.00 |
| Phosphoric acid | ad pH | 2.0 |
| Water | ad | 100.0 |

The coloration obtained was a glossy, black-blue, the hair being easy to comb and showing full texture and elasticity. The skin coloration was simple to remove by means of rubbing with a cotton cloth moistened with a stain remover.

### Example 12: (not according to the invention)

| | | |
|---|---|---|
| Acid Red 52 | | 0.50 % by wt. |
| D&C Orange 4 | | 0.25 |
| Benzyl alcohol | | 2.00 |
| Alcohol, den. | | 15.00 |
| Dimethicone copolyol | | 1.00 |
| Ceteareth-20 | | 3.00 |
| Sodium cetearyl sulfate | | 1.20 |
| Phosphoric acid | ad pH | 2.0 |
| Water | ad | 100.0 |

The coloration obtained was a very intensive, glossy red, the hair being easy to comb and showing full texture and elasticity. The skin coloration was simple to remove by means of rubbing with a cotton cloth moistened with a stain remover.

### Example 13: (not according to the invention)

| | | |
|---|---|---|
| Acid Red 52 | | 0.50 % by wt. |
| D&C Orange 4 | | 0.20 |
| Benzyl urea | | 2.00 |
| Alcohol, den. | | 15.00 |
| Dimethicone copolyol | | 1.00 |
| Sodium cetearyl sulfate | | 1.00 |
| Ceteareth-30 | | 5.00 |
| Lactic acid | | 4.50 |
| Water | ad | 100.0 pH 3.0 |

The coloration obtained was a very intensive, glossy red, the hair being easy to comb and showing full texture and elasticity. The skin coloration was simple to remove by means of rubbing with a cotton cloth moistened with a stain remover.

### Example 14:

| | | |
|---|---|---|
| Acid Violet 43 | | 0.30 % by wt. |
| Acid Black 1 | | 0.30 |
| D&C Orange 4 | | 0.20 |
| D&C Red 33 | | 0.20 |
| Alcohol, den. | | 15.00 |
| Benzyl alcohol | | 1.00 |
| Cetearyl alcohol | | 15.00 |
| Ceteareth-20 | | 5.00 |
| Lactic acid | | 4.50 |
| Dimethicone copolyol | | 1.00 |
| Perfume | | 0.20 |
| Phosphoric acid | ad pH | 2.0 |
| Water | ad | 100.0 |

The coloration obtained was a very intensive, glossy blue-black, the hair being easy to comb and showing full texture and elasticity. The skin coloration was simple to remove by means of rubbing with a cotton cloth moistened with a stain remover.

### Example 15:

| | | |
|---|---|---|
| Acid Violet 43 | | 0.30 % by wt. |
| Acid Black 1 | | 0.30 |
| D&C Orange 4 | | 0.20 |
| D&C Red 33 | | 0.20 |
| Cetearyl alcohol | | 11.25 |
| Ceteareth-20 | | 3.75 |
| Benzyloxyethanol | | 5.00 |
| Alcohol, den. | | 10.00 |
| Benzyl carbamate | | 1.70 |
| Hydroxyethyl cellulose | | 1.50 |
| Sodium hydroxide | | 0.25 |
| Dimethicone copolyol | | 1.00 |
| Phosphoric acid | ad pH | 2.00 |
| Water | ad | 100.0 |

The coloration obtained was a very intensive, glossy blue-black, the hair being easy to comb and showing full texture and elasticity. The skin coloration was simple to remove by means of rubbing with a cotton cloth moistened with a stain remover.

### Example 16:

| | | |
|---|---|---|
| Acid Red 52 | | 0.50 % by wt. |
| D&C Orange 4 | | 0.25 |
| Benzyl alcohol | | 2.00 |
| Alcohol, den. | | 15.00 |
| Dimethicone copolyol | | 1.00 |
| Cetearyl alcohol | | 12.00 |
| Ceteareth-20 | | 3.00 |
| Phosphoric acid | ad pH | 2.00 |
| Water | ad | 100.0 |

The coloration obtained was a very intensive, glossy red, the hair being easy to cc and showing full texture and elasticity. The skin coloration was simple to remove means of rubbing with a cotton cloth moistened with a stain remover.

### Example 17:

| | | |
|---|---|---|
| Acid Red 52 | | 0.50 % by wt. |
| D&C Orange 4 | | 0.25 |
| Benzyl urea | | 2.00 |
| Alcohol, den. | | 15.00 |
| Dimethicone copolyol | | 1.00 |
| Cetearyl alcohol | | 10.00 |
| Ceteareth-30 | | 3.00 |
| Lactic acid | | 4.50 |
| Water | ad | 100.0 pH 3.0 |

The coloration obtained was a very intensive, glossy red, the hair being easy to comb and showing full texture and elasticity. The skin coloration was simple to remove by means of rubbing with a cotton cloth moistened with a stain remover.

## Claims

1. Hair dyeing composition on aqueous basis in emulsion form **characterized in that** it comprises at least one direct-acting anionic dyestuff, an emulsifier selected from anionic, non-ionic, cationic, zwitterionic and amphoteric, and one or more fatty alcohols of the formula
R₁₃-OH
where R₁₃ is a saturated or unsatunted, branched or non-branched fatty acyl chain with 10 - 24 C atoms and has a pH in the range of 1 to 5.

2. Hair dyeing composition according to claim 1 **characterized in that** it has a pH-value in the range of 1,5 to 4.5.

3. Hair dyeing composition according to claim 1 and 2 **characterized in that** it has a pH-value in the range of 1.5 to 3.5.

4. Hair dyeing composition according to any of the preceding claims **characterized in that** the emulsifiers are of nonionic and/or anionic character.

5. Hair dyeing composition according to any of the preceding claims that it contains at least one direct acting anionic dye at a concentration of 0.005 - 5% by weight calculated to total composition.

6. Hair dyeing composition according to any of the preceding claims that it contains at least one direct acting anionic dye at a concentration of 0.01 % to 3.5 % by weight, calculated to the total composition.

7. Hair dyeing composition according to any of the preceding claims that it contains at least one direct acting anionic dye at a concentration of 0,1 % to 2.5% and more preferably 0.1 -1.0% by weight, calculated to the total composition.

8. Hair dyeing composition according to any of the preceding claims that it contains at least one direct acting anionic dye at a concentration of 0.1 -1.0% by weight, calculated to the total composition.

9. Hair dyeing composition according to any of the preceding claims that it contains emulsifier at a concentration of 0.1 10% by weight calculated to total composition.

10. Hair dyeing composition according to any of the preceding claims that it contains emulsifier at a concentration of 0.5 - 5% by weight calculated to total composition.

11. Hair dyeing emulsion composition according to any of the preceding claims that it contains fatty alcohol less than 20% by weight calculated to the total composition.

12. Hair dyeing composition according to one or more of the preceding claims, comprising at least one organic solvent as a penetration enhancer at a concentration of at least 5% by weight calculated to the total composition.

13. Hair dyeing composition according to one or more of the preceding claims, comprising at least one organic solvent as a penetration enhancer at a concentration of less than 35% by weight calculated to the total composition.

14. Hair dyeing emulsion composition according to any of the preceding claims that it contains organic or inorganic acids or their mixtures.

15. Hair dyeing emulsion composition according to any of the preceding claims that it has a viscosity of 1000 to 80000 mPa.s measured at 20°C with a Brookfield

16. Hair dyeing composition according to any of the preceding claims, comprising additionally one or more hair conditioning agents.

17. Hair dyeing emulsion composition according to any of the preceding claims that it shows lower skin coloring when tested under the conditions disclosed with the example 1 when compared to coloring agents containing acidic dyes thickened only with polymers and not in the emulsion form.

## Revendications

1. Composition pour teinture capillaire sur une base aqueuse sous forme d'émulsion **caractérisée en ce qu'**elle comprend au moins une matière colorante anionique à action directe, un émulsifiant de caractère choisi parmi anionique, non ionique, cationique, zwittérionique et amphotère et un ou plusieurs alcools gras de formule
R₁₃-OH
dans laquelle R₁₃ est une chaîne d'acyle gras ramifiée ou non ramifiée, saturée ou non saturée, avec 10 à 24 atomes de carbone, et possède un pH dans la gamme de 1 à 5.

2. Composition pour teinture capillaire selon la revendication 1, **caractérisée en ce qu'**elle possède une valeur de pH dans la gamme de 1,5 à 4,5.

3. Composition pour teinture capillaire selon les revendications 1 et 2, **caractérisée en ce qu'**elle possède une valeur de pH dans la gamme de 1,5 à 3,5.

4. Composition pour teinture capillaire selon l'une quelconque des revendications précédentes, **caractérisée en ce que** les émulsifiants sont de caractères non ionique et/ou anionique.

5. Composition pour teinture capillaire selon l'une quelconque des revendications précédentes, **caractérisée en ce qu'**elle contient au moins un colorant anionique à action directe à une concentration de 0,005 à 5 % en poids calculée par rapport à la composition totale.

6. Composition pour teinture capillaire selon l'une quelconque des revendications précédentes, **caractérisée en ce qu'**elle contient au moins un colorant anionique à action directe à une concentration de 0,01 % à 3,5 % en poids, calculée par rapport à la composition totale.

7. Composition pour teinture capillaire selon l'une quelconque des revendications précédentes, **caractérisée en ce qu'**elle contient au moins un colorant anionique à action directe à une concentration de 0,1 % à 2,5 % et de manière plus particulièrement préférée de 0,1 à 1,0 % en poids, calculée par rapport à la composition totale.

8. Composition pour teinture capillaire selon l'une quelconque des revendications précédentes, **caractérisée en ce qu'**elle contient au moins un colorant anionique à action directe à une concentration de 0,1 à 1,0 % en poids calculée par rapport à la composition totale.

9. Composition pour teinture capillaire selon l'une quelconque des revendications précédentes, **caractérisée en ce qu'**elle contient un émulsifiant à une concentration de 0,1 à 10 % en poids calculée par rapport à la composition totale.

10. Composition pour teinture capillaire selon l'une quelconque des revendications précédentes, **caractérisée en ce qu'**elle contient un émulsifiant à une concentration de 0,5 à 5 % en poids calculée par rapport à la composition totale.

11. Composition d'émulsion pour teinture capillaire selon l'une quelconque des revendications précédentes, **caractérisée en ce qu'**elle contient moins de 20 % en poids d'alcool gras calculé par rapport à la composition totale.

12. Composition pour teinture capillaire selon une ou plusieurs des revendications précédentes, comprenant au moins un solvant organique en tant qu'améliorateur de pénétration à une concentration d'au moins 5 % en poids calculée par rapport à la composition totale.

13. Composition pour teinture capillaire selon une ou plusieurs des revendications précédentes, comprenant au moins un solvant organique en tant qu'améliorateur de pénétration à une concentration inférieure à 35 % en poids calculée par rapport à la concentration totale.

14. Composition d'émulsion pour teinture capillaire selon l'une quelconque des revendications précédentes, **caractérisée en ce qu'**elle contient des acides organiques ou inorganiques ou leurs mélanges.

15. Composition d'émulsion pour teinture capillaire selon l'une quelconque des revendications précédentes, **caractérisée en ce qu'**elle possède une viscosité de 1 000 à 80 000 mPa.s mesurée à 20°C à l'aide d'un viscosimètre Brookfield.

16. Composition pour teinture capillaire selon l'une quelconque des revendications précédentes, comprenant en plus un ou plusieurs agents revitalisants capillaires.

17. Composition d'émulsion pour teinture capillaire selon l'une quelconque des revendications précédentes, **caractérisée en ce qu'**elle présente une coloration de la peau plus faible quand elle est testée dans les conditions décrites par l'exemple 1 quand on compare aux agents de coloration contenant des colorants acides n'étant épaissis qu'avec des polymères et n'étant pas sous la forme d'émulsion.

## Patentansprüche

1. Emulsionsförmiges Haarfärbemittel auf wässriger Basis, enthaltend mindestens einen direktziehenden anionischen Haarfarbstoff, einen anionischen, nichtionischen und/oder kationischen Emulgator und ein oder mehrere Fettalkohole der Formel R₁₃-OH, worin R₁₃ eine gesättigte oder ungesättigte, verzeigte oder nicht-verzweigte fettige Alkylkette mit 10 bis 24 C-Atomen ist, das einen pH-Wert zwischen 1 bis 5 aufweist.

2. Haarfärbemittel nach Anspruch 1, das einen pH-Wert zwischen 1,5 bis 4,5 aufweist.

3. Haarfärbemittel nach Anspruch 1 und 2, das einen pH-Wert zwischen 1,5 und 3,5 aufweist.

4. Haarfärbemittel nach einem der Ansprüche 1 bis 3, **dadurch gekennzeichnet, dass** der Emulgator einen nichtionischen und/oder anionischen Charakter aufweist.

5. Haarfärbemittel nach einem der vorangegangenen Ansprüche, **dadurch gekennzeichnet, dass** es mindestens einen direktziehenden anionischen Farbstoff in einer Konzentration von 0,005 bis 5 Gew.-%, berechnet auf die Gesamtzusammensetzung, enthält.

6. Haarfärbemittel nach einem der vorangegangenen Ansprüche, **dadurch gekennzeichnet, dass** es mindestens einen direktziehenden anionischen Farbstoff in einer Konzentration von 0,01 bis 3,5 Gew.-%, berechnet auf die Gesamtzusammensetzung, enthält.

7. Haarfärbemittel nach einem der vorangegangenen Ansprüche, **dadurch gekennzeichnet, dass** es mindestens einen direktziehenden anionischen Farbstoff in einer Konzentration von 0,1 bis 2,5 Gew.%, bevorzugt 0,1 bis 1 Gew.-%, berechnet auf die Gesamtzusammensetzung, enthält.

8. Haarfärbemittel nach einem der vorangegangenen Ansprüche, **dadurch gekennzeichnet, dass** es mindestens einen direktziehenden anionischen Farbstoff in einer Konzentration von 0,1 bis 1 Gew.-%, berechnet auf die Gesamtzusammensetzung, enthält.

9. Haarfärbemittel nach einem der vorangegangenen Ansprüche. **dadurch gekennzeichnet, dass** es Emulgatoren in einer Konzentration von 0,1 bis 10 Gew.-%, berechnet auf die Gesamtzusammensetzung, enthält.

10. Haarfärbemittel nach einem der vorangegangenen Ansprüche. **dadurch gekennzeichnet, dass** es Emulgatoren in einer Konzentration von 0,5 bis 5 Gew.-%, berechnet auf die Gesamtzusammensetzung, enthält.

11. Emulsionsartiges Haarfärbemittel nach einem der vorangegangenen Ansprüche, enthaltend Fettalkohole in einer Konzentration von weniger als 20 Gew.-%, berechnet auf die Gesamtzusammensetzung.

12. Haarfärbemittel nach einem oder mehreren der vorangegangenen Ansprüche, enthaltend mindestens ein organisches Lösungsmittel als Penetrationsverstärker in einer Konzentration von weniger als 5 Gew.-%, bezogen auf die Gesamtzusammensetzung.

13. Haarfärbemittel nach einem oder mehreren der vorangegangenen Ansprüche, enthaltend mindestens ein organisches Lösungsmittel als Penetrationsverstärker in einer Konzentration von weniger als 35 Gew.-%, bezogen auf die Gesamtzusammensetzung.

14. Emulsionsartiges Haarfärbemittel nach einem der vorangegangenen Ansprüche, **dadurch gekennzeichnet, dass** es organische oder anorganische Säuren oder Mischungen davon enthält.

15. Emulsionsartiges Haarfärbemittel nach einem der vorangegangenen Ansprüche, **dadurch gekennzeichnet, dass** es eine Viskosität von 1000 zu 80000 mPa.s, gemessen mit einem Brookfield Viskosimeter bei 20°C, aufweist.

16. Haarfärbemittel nach einem der vorangegangenen Ansprüche, enthaltend zusätzlich eines oder mehrere haarkonditionierende Substanzen.

17. Emulsionsartiges Haarfärbemittel nach einem der vorangegangenen Ansprüche, **dadurch gekennzeichnet, dass** es unter den in Beispiel 1 aufgezeigten Bedingungen eine geringer Hautfärbung aufweist, als Haarfärbemittel, die saure Farbstoffe enthalten und nur mit Polymeren verdickt werden und nicht in Emulsionsform vorliegen.
